(19) Europäisches Patentamt
European Patent Office
Office européen des brevets

(11) Numéro de publication : **0 141 684 B1**

(12) # FASCICULE DE BREVET EUROPÉEN

(45) Date de publication du fascicule du brevet : **30.09.87**

(51) Int. Cl.⁴ : **C 07 J 31/00**, A 61 K 31/57

(21) Numéro de dépôt : **84401553.7**

(22) Date de dépôt : **24.07.84**

(54) **17-oxo-21-thioesters d'hydrocortisone, leur préparation et leurs applications comme médicament.**

(30) Priorité : **26.08.83 FR 8313777**

(43) Date de publication de la demande : **15.05.85 Bulletin 85/20**

(45) Mention de la délivrance du brevet : **30.09.87 Bulletin 87/40**

(84) Etats contractants désignés : **AT BE CH DE GB IT LI LU NL SE**

(56) Documents cités :
**EP-A- 0 072 268**
**FR-A- 2 081 395**
**FR-A- 2 231 374**
**FR-A- 2 529 894**
**Burger's Medicinal Chemistry, 4ème ed. Vol. III, p. 1215, 1285**
**Eur.J. Rheumatol. Inflamm. 1978 T1 No. 3 pp. 204-211**
**FED. Proceedings, Vol. 41 No. 9 - 2588-2595**
**Handbook of Inflammation Vol. 5, Chapt.9, pages 371-373**
**Nature, vol. 287 (11 sept. 1980) pages 147-149**
**PNAS USA vol. 74 No. 4 pp. 1716-1720**
**Cardiovascular Pharmacology of the prostaglandin Raven Press New York 1982, pages 14-22**
**Le dossier contient des informations techniques présentées postérieurement au dépôt de la demande et ne figurant pas dans le présent fascicule.**

(73) Titulaire : **JOUVEINAL S.A.**
**Tour Maine Montparnasse 33 Avenue du Maine**
**F-75755 Paris Cedex 15 (FR)**

(72) Inventeur : **Aubard, Gilbert Gustave**
**7 Chemin de la Savetière**
**F-91120 Palaiseau (FR)**
Inventeur : **Grouhel, Agnès Geneviève**
**2, rue des Peupliers**
**F-92190 Meudon (FR)**
Inventeur : **Junien, Jean-Louis**
**85 Boulevard Suchet**
**F-75016 Paris (FR)**
Inventeur : **Roux, Claude Paul Joseph**
**32 Square Montsouris**
**F-75014 Paris (FR)**
Inventeur : **Torossian, Diéran Robert**
**11, rue Jean Bastard**
**F-92340 Bourg-la-Reine (FR)**

(74) Mandataire : **Bourgognon, Jean-Marie et al**
**Cabinet Flechner 22, Avenue de Friedland**
**F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue St-Denis, 75001 Paris, France

**Description**

Au brevet belge n° 893 957, on décrit des composés de formule :

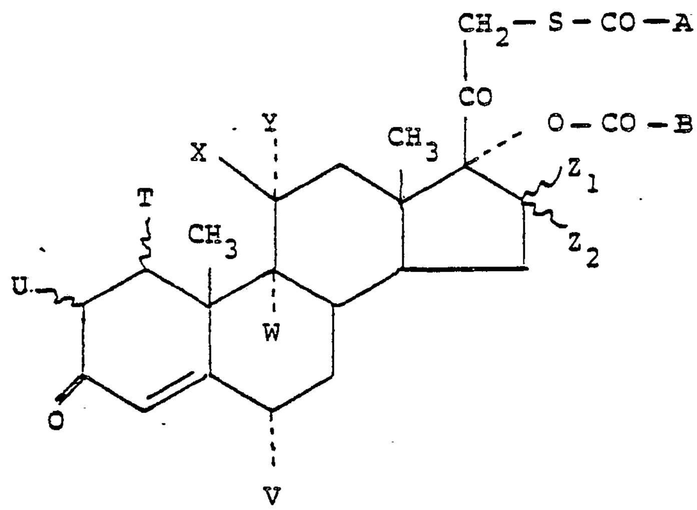

dans laquelle A et B sont chacun, indépendamment l'un de l'autre, un radical alcoyle linéaire ou ramifié ayant de 1 à 6 atomes de carbone ou un radical phényle éventuellement mono- ou plurisubstitué par des radicaux alcoyle ayant de 1 à 6 atomes de carbone, alcoxy ayant de 1 à 6 atomes de carbone ou halogène, T et U sont, indépendamment l'un de l'autre, des atomes d'hydrogène ou forment ensemble une double liaison, V est un atome d'hydrogène ou un radical méthyle en position α, W est un atome d'hydrogène ou un atome d'halogène en position α, X est un radical hydroxy en position β et Y est un atome d'hydrogène ou X et Y peuvent représenter ensemble un atome d'oxygène, et $Z_1$ est un atome d'hydrogène, un radical méthyle en position α ou β, alors que $Z_2$ est un atome d'hydrogène ou $Z_1$ et $Z_2$ forment ensemble un radical méthylène.

Ces composés se sont révélés posséder des propriétés anti-inflammatoires supérieures, parfois de plus de 100 fois, à celles de stéroïdes anti-inflammatoires locaux dont les activités sont reconnues être les meilleures pour les produits de cette classe.

Or on a maintenant trouvé que parmi ces composés de valeur, deux d'entre eux, le S-21-thioacétate 17-butyrate de 3,20-dione 11β-hydroxy-prègn-4-ène et S-21-thiopropionate 17-butyrate de 3,20-dione 11β-hydroxy-prègn-4-ène ont des propriétés anti-inflammatoires locales particulières et inattendues très supérieures à celles des meilleurs composés du brevet belge.

L'invention vise donc, à titre d'invention de sélection, les deux composés mentionnés ci-dessus utiles notamment comme médicament anti-inflammatoire, ainsi que leur procédé de préparation.

Le procédé de préparation des produits de la présente invention consiste essentiellement à faire réagir un 21-sulfonate de stéroïde de formule :

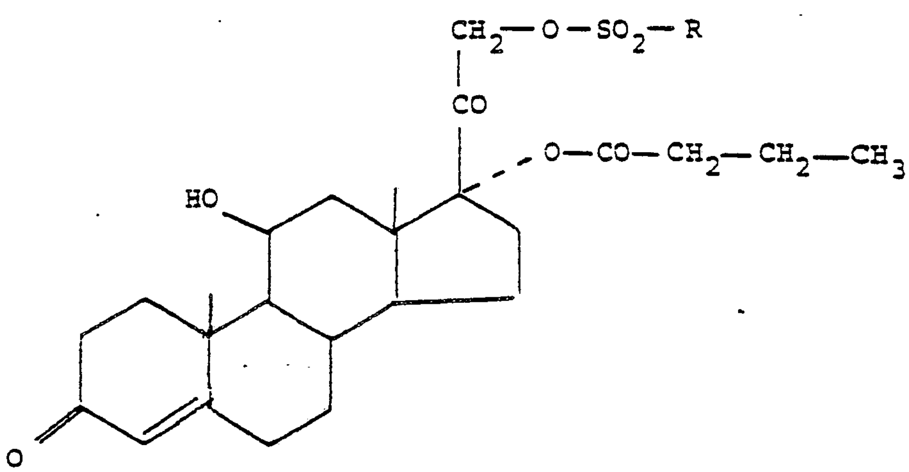

dans laquelle R correspond à un radical alcoyle, ayant notamment de 1 à 6 atomes de carbone, avec un thiocarboxylate de métal alcalin de formule :

$$M{-}S{-}CO{-}R_1$$

dans laquelle M représente un atome de métal alcalin et $R_1$ est méthyle ou éthyle.

Ces produits de départ sont connus en soi et leur préparation est réalisée par des méthodes déjà décrites (cf. brevet belge n° 893 957).

La réaction de condensation entre ces produits s'effectue dans un solvant aprotique de nature appropriée comme l'acétone ou l'hexaméthylphosphorotriamide (H.M.P.T.).

Le produit brut obtenu est généralement purifié par chromatographie sur colonne, puis recristallisé dans un alcool, notamment un alcool de bas poids moléculaire, pour obtenir un produit de pureté convenable pour une utilisation thérapeutique.

La pureté des produits obtenus est déterminée par des méthodes connues de l'homme de l'art : chromatographie sur couches minces, spectrographies ultra-violette et infrarouge, analyse élémentaire.

A titre d'illustration dans les exemples qui suivent les produits de l'invention sont caractérisés par leurs points de fusion non corrigés déterminés sur un appareil « FP 1 Mettler » et par les principaux signaux de leurs spectres de résonance magnétiques nucléaires du proton (R.M.N.). Ces spectres ont été enregistrés en solution dans le deutérochloroforme sur un appareil de 60 MHz et les déplacements chimiques sont exprimés en p.p.m. par rapport au tétraméthylsilane pris comme référence.

## Exemple 1

S-21-thioacétate, 17-butyrate de 3,20-dione, 11β hydroxy-prègn-4-ène (ou d'hydrocortisone).

Dans un réacteur on introduit 10,0 g (0,129 mol) d'acide S-thioacétique et 220 ml d'hexaméthylphosphorotriamide (H.M.P.T.).

Sous agitation, à une température voisine de 20 °C, on introduit 27,7 ml d'une solution méthanolique de méthylate de sodium 4,65 N (0,129 mol). Après introduction, la solution beige est maintenue une heure sous agitation à la température ambiante.

En 10 minutes on introduit une solution de 44,0 g (0,086 mol) de 21-mésylate, 17-butyrate de cortisol dans 440 ml d'H.M.P.T.

La solution est maintenue 2,5 h sous agitation à la température ambiante. La solution orangée est précipitée dans 8 litres d'eau glacée. L'insoluble qui se forme est filtré puis repris par l'éther éthylique.

La solution éthérée est extraite à deux reprises par 250 ml de solution d'hydroxyde de sodium N puis 3 fois par 500 ml de solution saturée de chlorure de sodium. Après séchage de la phase éthérée le solvant est éliminé par distillation.

Le résidu (39,0 g) est purifié par chromatographie sur colonne à l'aide de 1,00 kg de « Florisil »® (marque déposée).

L'élution par un mélange dichlorométhane-acétone 95-5 (v/v) permet de recueillir 21,0 g de produit purifié.

Ce produit est finalement recristallisé dans 170 ml de mélange méthanol-eau 8-2 (v/v).

Poids = 19,0 g.

Rendement = 44,3 %.

Point de fusion = 130 °C.

R.M.N. = 1,00 (s, 18 $CH_3$) ; 1,48 (s, 19 $CH_3$) ;
1,70 (m, 11 βOH) ; 2,35 (s, $CH_3$ ester en 21) ;
3,8 (s, 21 $CH_2$) ; 4,55 (m, 11 CH) ; 5,70 (s, 4 CH).

## Exemple 2

S-21-thiopropionate, 17-butyrate de 3,20-dione-11β hydroxy-prègn-4-ène (ou d'hydrocortisone).

D'une façon identique à celle décrite dans l'exemple 1, à partir de 225,0 g (0,44 mol) de 21-mésylate 17-butyrate de cortisol, de 59,6 g (0,66 mol) d'acide S-thiopropionique et de 142,0 ml de solution méthanolique de méthylate de sodium 4,65 N (0,66 mol), on obtient 200,0 g de produit purifié qui sont finalement recristallisés dans 450,0 ml de méthanol.

Poids = 95,0 g.

Rendement = 42,8 %.

Point de fusion = 140,5 °C.

R.M.N. = 1,00 (s, 18 $CH_3$) ; 1,50 (s, 19 $CH_3$) ; 1,70 (m, 11 βOH) ; 3,90 (s, 21 $CH_2$) ; 4,50 (m, 11 CH) ; 5,75 (s, 4 CH).

La puissante activité de certains stéroïdes les rend particulièrement utiles pour le traitement d'affections diverses en administration par voie générale et/ou locale. Ces traitements sont le plus souvent délicats et doivent être conduits avec précautions sous surveillance médicale.

Il est fréquent que l'activité anti-inflammatoire de ces structures soit accompagnée d'activités secondaires parallèles qui induisent des effets néfastes parfois graves et irréversibles. Ces effets sont le plus souvent causés par la nature même du stéroïde. Pour la classe des produits d'activité locale, ces effets et les précautions nécessaires à leur utilisation ont été souvent décrits et récemment par A. Fritz (Ann. of Allergy, vol. 50, n° 2, page 68, 1983).

La dissociation de ces activités est donc un critère de choix majeur pour l'utilisation thérapeutique de

tels produits. A cet égard, les produits de la présente invention montrent, d'une façon imprévisible, une activité anti-inflammatoire remarquable pratiquement dénuée d'effets secondaires.

Des études classiques chez l'homme et l'animal permettent de déterminer les qualités des produits d'activité anti-inflammatoire locale.

Détermination de l'activité anti-inflammatoire locale

Chez l'homme, il est fait couramment appel au test de vasoconstriction, appelé aussi test de blanchiment selon Mac Kenzie et Stoughton (Arch. Dermatol. vol. 86, page 608, 1962). Ce test est reconnu refléter fidèlement l'activité thérapeutique d'un produit.

Chez l'animal, les tests les plus utilisés sont le test du granulome au pellet de coton d'après C.A. Winter ou Meïer (J. Am. Pharm. Ass., vol. 46, n° 9, page 515, 1957 et Experientia, vol. 6, page 608, 1962) et le test de l'érythème à l'huile de croton selon la méthode décrite par Tonelli (Endocrinology, vol. 77, page 625, 1965).

Ce dernier test conduit à des résultats comparables à ceux du test de vasoconstriction (O.J. Lorenzetti — Curr. Therap. Res., vol. 25, n° 1, page 92, 1979), par conséquence il est, chez l'animal, le plus représentatif de l'activité thérapeutique d'un produit chez l'homme.

O. J. Lorenzetti et N. Bodor (réf. déjà citée et J. Med. Chem., vol. 26, page 318, 1983) présentent comparativement au test de vasoconstriction les résultats obtenus avec les tests de l'érythème et celui du granulome. L'exemple du 17-butyrate d'hydrocortisone, stéroïde réputé et commercialisé pour son activité anti-inflammatoire locale [Locoïd (marque déposée)] a conduit la demanderesse à préférer pour l'étude des produits de la présente invention le test de l'érythème selon Tonelli.

En effet, d'après N. Bodor, ce produit ne présente qu'une faible activité dans le test du granulome, ce qui le place dans la classe des produits peu ou pas actifs, alors que les résultats du test à l'érythème rapportés par O.J. Lorenzetti, montrent une bonne similitude d'activité vis-à-vis du test de vasoconstriction.

Détermination des effets secondaires-activité systémique.

Ces effets ont été évalués par l'intermédiaire de leur activité thymolytique.

L'administration répétée de produits d'activité systémique provoque un affaiblissement des systèmes de défense de l'organisme. Cette diminution se traduit, particulièrement chez les jeunes animaux, par une régression de poids de deux organes du système réticulo-endothélial : la rate et le thymus, ce dernier étant le plus sensible à cet effet.

Les résultats obtenus par cette étude permettent de calculer le rapport d'activité :

$$\frac{\text{activité locale anti-inflammatoire}}{\text{activité systémique}}$$

rapport d'autant plus favorable et significatif d'une dissociation d'activités que le résultat obtenu se traduit par un chiffre faible.

Les propriétés particulièrement intéressantes des produits de la présente invention ont été étudiées comparativement aux structures suivantes :

-S-21-propionate 17-butyrate d'hydrocortisone
-S-21-acétate 17-valérate de prednisolone
-S-21-thiopivalate d'hydrocortisone
-S-21-thioacétate 17-valérate de prednisolone
-S-21-thioacétate 17-valérate de 6-α-méthylprednisolone*
-S-21-thiopropionate 17-acétate de bétaméthasone*
-S-21-thioacétate 17-propionate de bétaméthasone*
-S-21-thiopropionate 17-propionate de bétaméthasone*
-S-21-thiotertiobutylacetate 17-acétate de dexaméthasone*
-S-21-thiopropionate 17-propionate de béclométhasone*

Dans cette étude, le S-21-acétate d'hydrocortisone a été utilisé comme substance de référence du présent mémoire.

On donne ci-dessous les protocoles opératoires et les résultats des études pharmacologiques.

D'une façon générale, les composés suivant l'invention, administrés à l'animal par des voies compatibles à leurs propriétés d'insolubilité dans l'eau, ne présentent pas de phénomènes de toxicité aiguë.

*Produits qui correspondent dans l'ordre respectivement aux exemples 7, 9, 10, 11, 14 et 15 du brevet belge n° 893 957.

I - Techniques

I.1. Activité anti-inflammatoire locale

Cette étude a été réalisée selon une technique dérivée de celle de Tonelli (Endocrinology, vol. 77, page 625, 1965).

Le test consiste à provoquer une inflammation de l'oreille chez la souris à l'aide d'une solution irritante à base d'huile de croton.

L'addition d'une substance anti-inflammatoire locale à cette solution a pour conséquence de provoquer une inflammation inférieure à celle des animaux témoins, c'est-à-dire traités par la solution irritante seule.

Les résultats de l'étude sont exprimés en $CE_{50}$. Cette expression est la concentration en % p/v du produit en solution capable d'entraîner une diminution de 50 % de l'inflammation par rapport à celle provoquée chez les animaux témoins.

I.2. Activité systémique

Cette activité a été déterminée au moyen de l'étude de l'activité des produits sur la régression du poids des thymus des animaux.

Les produits à étudier en suspension dans la gomme arabique à 5 % p/v sont administrés quotidiennement par voie orale et ce pendant quatre jours consécutifs à de jeunes rats de souche Wistar. Un lot témoin d'animaux reçoit dans les mêmes conditions un volume identique de l'excipient.

Quatre vingt-seize heures après la première administration, les animaux sont sacrifiés, leurs thymus prélevés et pesés immédiatement.

Les résultats de l'étude sont exprimés en $DE_{50}$, expression qui correspond à la dose en milligrammes par jour de traitement capable de provoquer une diminution de 50 % du poids des thymus pour un kilogramme d'animal par rapport aux animaux du lot témoin $mg \times kg^{-1} \times d^{-1}$

II - Résultats et commentaires

II.1. Résultats

Ces résultats pour les deux tests précédemment décrits sont rapportés dans le tableau I qui suit.

Tableau I

| | Produit étudié | Activité anti-inflammatoire | | Activité systémique | |
|---|---|---|---|---|---|
| | | $CE_{50}$ % p/v | Activité/réf. 21 act.hydro | $DE_{50}$ $mg \times kg^{-1} \times d^{-1}$ | Activité/réf. 21 act.hydro |
| Réf. | S-21-acétate d'hydrocortisone | 0,30 | 1 | 14 | 1 |
| Produits de comparaison | S-21-propionate 17-butyrate d'hydrocortisone | 0,0047 | 63,8 | 7,2 | 1,95 |
| | S-21-acétate 17-valérate de prednisolone | 0,0018 | 167 | 9,0 | 1,60 |
| | S-21-thiopivalate d'hydrocortisone | 2,5 | 0,12 | 3 600 | 0,0038 |
| | S-21-thioacétate 17-valérate de prednisolone | 0,008 | 37,5 | 58 | 0,24 |
| | S-21-thioacétate 17-valérate de 6-α-méthyl-prednisolone | 0,5 | 0,6 | 43 | 0,325 |
| | S-21-thiopropionate 17-acétate de bêtaméthasone | 0,012 | 25,0 | 2,4 | 5,83 |
| | S-21-thioacétate 17-propionate de bêtaméthasone | 0,010 | 30,0 | 2,5 | 5,60 |
| | S-21-thiopropionate 17-propionate de bêtaméthasone | 0,0013 | 231 | 3,0 | 4,66 |

Tableau I (Suite)

| | Produit étudié | Activité anti-inflammatoire | | Activité systémique | |
|---|---|---|---|---|---|
| | | $CE_{50}$ % p/v | Activité/réf. 21 act.hydro | $DE_{50}$ $mg \times kg^{-1} \times d^{-1}$ | Activité/réf. 21 act.hydro |
| Produits de comparaison | S-21-thio-t-butylacétate 17-acétate de dexaméthasone | 0,0092 | 32,6 | 3,0 | 4,67 |
| | S-21-thiopropionate 17-propionate de béclométhasone | 0,0175 | 17,1 | 19 | 0,74 |
| Produits de l'invention | Exemple 1 : S-21-thioacétate, 17-butyrate d'hydrocortisone | 0,0082 | 36,6 | 350 | 0,04 |
| | Exemple 2 : S-21-thiopropionate, 17-butyrate d'hydrocortisone | 0,021 | 14,3 | > 1 450 | < 0,0096 |

II.2. Exploitation des résultats

Les résultats présentés dans le tableau précédent permettent de déterminer un rapport :

$$\frac{\text{activité anti-inflammatoire locale}}{\text{activité systémique}}$$

La valeur de ce rapport traduit la dissociation des activités et est inversement proportionnelle à l'importance de la dissociation, c'est-à-dire que plus cette valeur est faible, plus la dissociation est grande.

Cette valeur a été calculée d'après l'expression :

$$R = \frac{CE_{50} \text{ activité anti-inflammatoire locale}}{DE_{50} \text{ activité systémique}} \times 10^4$$

Une comparaison avec le 21-acétate d'hydrocortisone est également présentée dans le tableau II suivant.

Tableau II

| | Produit étudié | R | R 21-acet.hydroc. / R produit étudié |
|---|---|---|---|
| Réf. | S-21-acétate d'hydrocortisone | 214 | 1 |
| Produits de comparaison | S-21-propionate 17-butyrate d'hydrocortisone | 6,52 | 32,8 |
| | S-21-acétate 17-valérate de prednisolone | 2,0 | 107,0 |
| | S-21-thiopivalate d'hydrocortisone | 6,90 | 31,0 |
| | S-21-thioacétate 17-valérate de prednisolone | 1,38 | 155 |
| | S-21-thioacétate 17-valérate de 6-α-méthylprednisolone | 116,3 | 1,85 |
| | S-21-thiopropionate 17-acétate de bêtaméthasone | 50,0 | 4,30 |
| | S-21-thioacétate 17-propionate de bêtaméthasone | 40,0 | 5,35 |
| | S-21-thiopropionate 17-propionate de bêtaméthasone | 4,33 | 49,40 |
| | S-21-thio-t-butylacétate 17-acétate de dexaméthasone | 30,7 | 7,0 |
| | S-21-thiopropionate 17-propionate de béclométhasone | 9,21 | 23,20 |

Tableau II (Suite)

| | Produit étudié | R | R 21-acet.hydroc. / R produit étudié |
|---|---|---|---|
| Produits de l'invention | Exemple 1 : S-21-thioacétate, 17-butyrate d'hydrocortisone | 0,23 | 930 |
| | Exemple 2 : S-21-thiopropionate, 17-butyrate d'hydrocortisone | 0,14 | 1 528 |

Les dérivés de la présente invention montrent, de façon générale, une activité anti-inflammatoire intéressante. La valeur de cette activité est dans l'ensemble comparable à celle des S-21-thio 17-oxo diesters de stéroïdes utilisés comme substance de comparaison et notamment pour des dérivés semblables en série bêtaméthasone, béclométhasone et dexaméthasone qui sont considérés comme des structures de base de puissante activité.

Toutefois les produits de l'invention ont une activité sensiblement inférieure aux diesters oxygénés étudiés.

Hormis pour le S-21-thiopivalate d'hydrocortisone, les produits de l'invention montrent, de façon surprenante, une spectaculaire diminution d'activité systémique par rapport aux produits de comparaison étudiés. En effet il est constaté que, dans cet essai, le produit de l'exemple 1, qui donne les moins bons résultats, parmi les deux produits sélectionnés, possède cependant une activité 6 fois inférieure au S-21-thioacétate 17-valérate de prednisolone, qui est le meilleur dans la série des produits de comparaison.

Le rapport de dissociation de ces activités montre, de façon indiscutable, la supériorité des produits de l'invention par rapport aux autres produits étudiés. Dans le cas particulier du produit de l'exemple 2, la dissociation des activités est plus de 45 fois supérieure à celle de son homologue, le 21-propionate 17-butyrate d'hydrocortisone produit du brevet français No. 2 421 480, alors qu'en série prednisolone le S-21-thioacétate 17-valérate ne montre qu'une dissociation environ 1,5 fois supérieure à celle de son homologue oxygéné (comparaison des 2ème et 4ème produits de comparaison).

Ces propriétés surprenantes démontrent la supériorité remarquable des produits de l'invention et leur intérêt comme agents anti-inflammatoires.

Les produits de la présente invention peuvent être administrés par voie générale et locale sous forme de composition pharmaceutique adaptée à leur utilisation. De même, ces propriétés permettent d'envisager des quantités d'agent actif de 0,1 à 100 mg par unité de dosage et journellement les agents actifs peuvent être administrés en quantités comprises entre 0,1 et 500 mg selon la nature et le site de l'affection à traiter.

A titre de composition administrable par voie générale il est retenu des formes solides ou liquides, telles que des solutions et des suspensions, injectables ou non, des comprimés, capsules, granulés et gélules.

Par voie locale, les compositions préférées sont les pommades, les onguents, les crèmes, des émulsions, des lotions, des gouttes, des lavements, des suppositoires, des ovules, des instillations et des aérosols.

Il va de soi que ces compositions pharmaceutiques peuvent contenir d'autres principes actifs compatibles avec les produits de l'invention comme, sans que cette énumération soit limitative, des agents de conservation, des agents bactériostatiques, des antibiotiques, des agents antimycotiques et des anesthésiques locaux.

Pour les compositions pharmaceutiques dans lesquelles les produits de l'invention se trouvent à l'état de suspension, il est avantageux d'utiliser des principes actifs sous forme micronisée d'une granulométrie moyenne de 5 µm. Dans certains cas particuliers, notamment pour les formes aérosols, il est nécessaire d'utiliser le principe actif sous forme micronisée de granulométrie voisine de 2 microns.

Ces compositions pharmaceutiques sont préparées selon des formulations et des conditions bien connues de l'homme de l'art et en respect des normes de l'industrie pharmaceutique.

Les propriétés des produits de la présente invention sont utiles pour le traitement d'affections inflammatoires d'origines les plus diverses.

Leur action est particulièrement utile pour le traitement des inflammations des muqueuses de la sphère O.R.L. et des bronches, notamment dans le traitement de l'asthme.

Ils sont également utiles pour le traitement des affections de la peau, comme par exemple l'eczéma et le psoriasis. Par ailleurs, leur action est bénéfique dans le traitement des affections rectales et du côlon.

Des affections internes, d'origines diverses comme les arthrites, les polyarthrites et diverses maladies d'origine allergique peuvent être traitées par les produits de la présente invention.

**Revendications** (pour les Etats contractants : BE, CH, DE, GB, LI, IT, LU, NL, SE)

1. Le S-21-thioacétate 17-butyrate de 3,20-dione 11β-hydroxy-prègn-4-ène.

2. Le S-21-thiopropionate 17-butyrate de 3,20-dione 11β-hydroxy-prègn-4-ène.

3. Procédé de préparation du S-21-thioacétate 17-butyrate de 3,20-dione 11β-hydroxy-prègn-4-ène et du S-21-thiopropionate 17-butyrate de 3,20-dione 11β-hydroxy-prègn-4-ène, caractérisé en ce qu'il consiste à faire réagir un 21-sulfonate de stéroïde de formule :

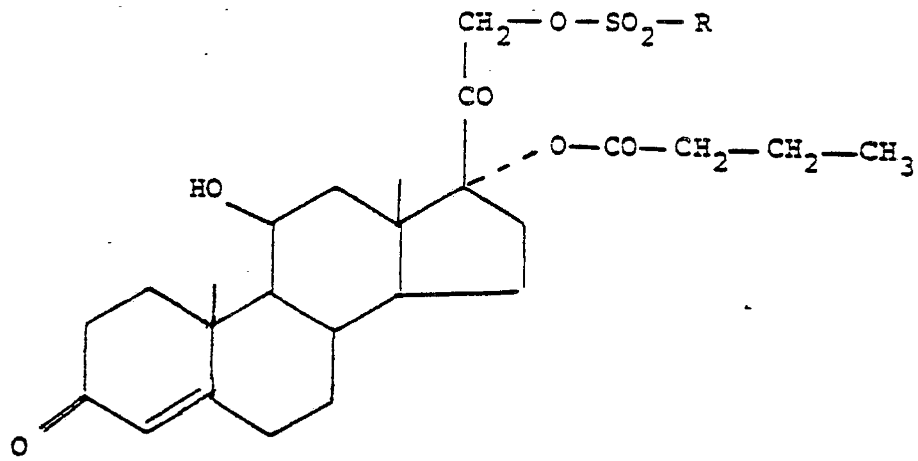

dans laquelle R est alcoyle inférieur sur un thiocarboxylate de métal alcalin de formule :

$$M—S—CO—R_1$$

dans laquelle M est un atome de métal alcalin et $R_1$ est méthyle ou éthyle.

4. Médicament notamment anti-inflammatoire, caractérisé en ce qu'il compend l'un des composés des revendications 1 et 2.

**Revendications** (pour l'Etat contractant AT)

1. Procédé de préparation du S-21-thioacétate 17-butyrate de 3,20-dione 11β-hydroxy-prègn-4-ène et du S-21-thiopropionate 17-butyrate de 3,20-dione 11β-hydroxy-prègn-4-ène, caractérisé en ce qu'il consiste à faire réagir un 21-sulfonate de stéroïde de formule :

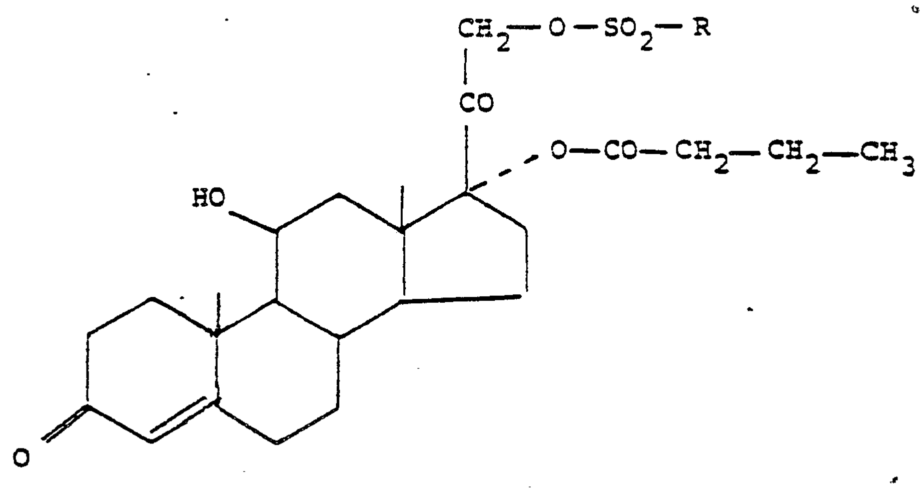

dans laquelle R est alcoyle inférieur sur un thiocarboxylate de métal alcalin de formule :

$$M—S—CO—R_1$$

dans laquelle M est un atome de métal alcalin et $R_1$ est méthyle ou éthyle.

2. Procédé de préparation d'une composition pharmaceutique, caractérisé en ce qu'il consiste à mélanger du S-21-thioacétate 17-butyrate de 3,20-dione 11-hydroxy-prègn-4-ène ou du S-21-thiopropionate 17-butyrate de 3,20-dione 11β-hydroxy-prègn-4-ène, à un véhicule ou excipient pharmaceutique.

**Claims** (for the Contracting States : BE, CH, DE, GB, IT, LI, LU, NL, SE)

1. 3,20-dione 11β-hydroxy-pregn-4-ene S-21 thioacetate-17-butyrate.
2. 3,20-dione 11β-hydroxy-pregn-4-ene S-21 thiopropionate-17-butyrate.
3. Process for the preparation of 3,20-dione 11β-hydroxy-pregn-4-ene S-21-thioacetate-17-butyrate and 3,20-dione 11β-hydroxy-pregn-4-ene S-21-thiopropionate-17-butyrate, characterized in that it comprises reacting a 21-sulphonate steroid of formula :

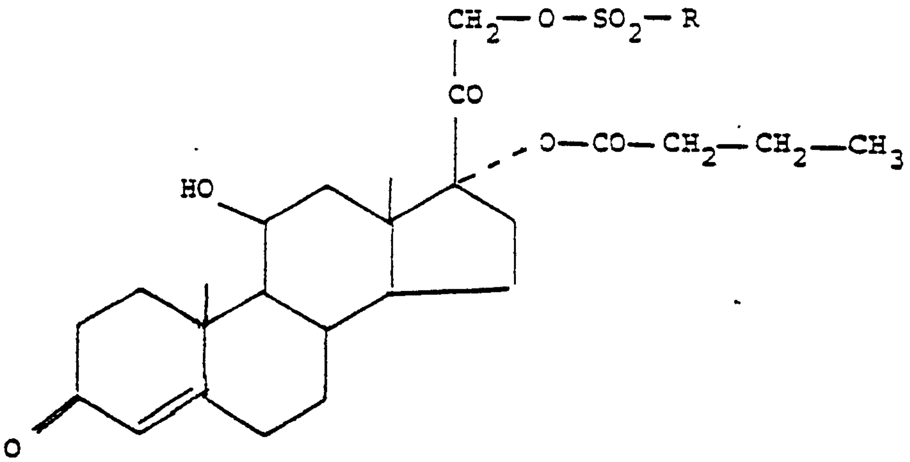

in which R is a lower alkyl with an alkali metal thiocarboxylate of formula :

$$M—S—CO—R_1$$

in which M is an alkali metal atom and $R_1$ methyl or ethyl.

4. Drug, in particular anti-inflammatory drug, characterized in that it comprises one of the compounds of claims 1 and 2.

**Claims** (for the Contracting State AT)

1. Process for the preparation of 3,20-dione 11β-hydroxy-pregn-4-ene S-21-thioacetate-17-butyrate and 3,20-dione 11β-hydroxy-pregn-4-ene S-21-thiopropionate-17-butyrate, characterized in that it comprises reacting a 21-sulphonate steroid of formula :

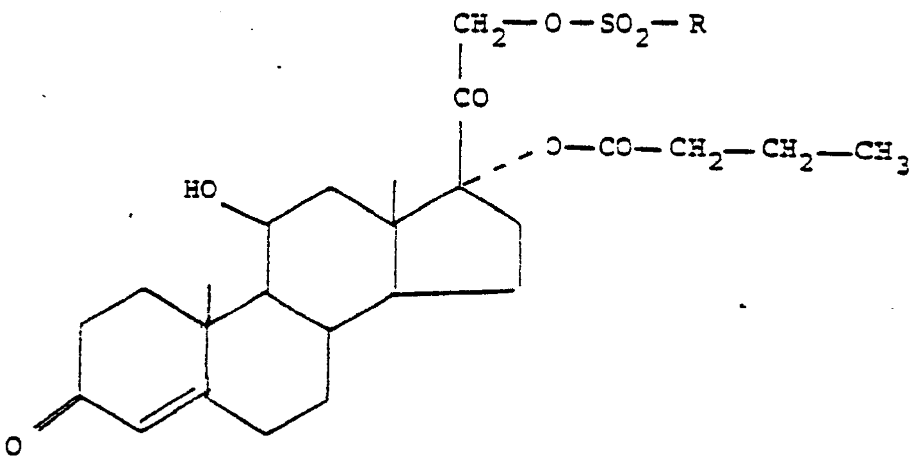

in which R is a lower alkyl with an alkali metal thiocarboxylate of formula :

$$M—S—CO—R_1$$

in which M is an alkali metal atom and $R_1$ methyl or ethyl.

2. Process for the preparation of a pharmaceutical composition, characterized in that it comprises mixing 3,20-dione 11β-hydroxy-pregn-4-ene S-21-thioacetate-17-butyrate or 3,20-dione 11β-hydroxy-pregn-4-ene S-21-thiopropionate-17-butyrate with a pharmaceutical excipient or vehicle.

**Patentansprüche** (für die Vertragsstaaten : BE, CH, DE, GB, IT, LI, LU, NL, SE)

1. Das S-21-Thioacetat-17-butyrat von 11β-Hydroxy-4-pregnen-3,20-dion.
2. Das S-21-Thiopropionat-17-butyrat von 11β-Hydroxy-4-pregnen-3,20-dion.
3. Verfahren zur Herstellung des S-21-Thioacetat-17-butyrat von 11β-Hydroxy-4-pregnen-3,20-dion und des S-21-Thiopropionat-17-butyrat von 11β-Hydroxy-4-pregnen-3,20-dion, dadurch gekennzeichnet, daß man ein 21-Sulfonat des Steroides der Formel

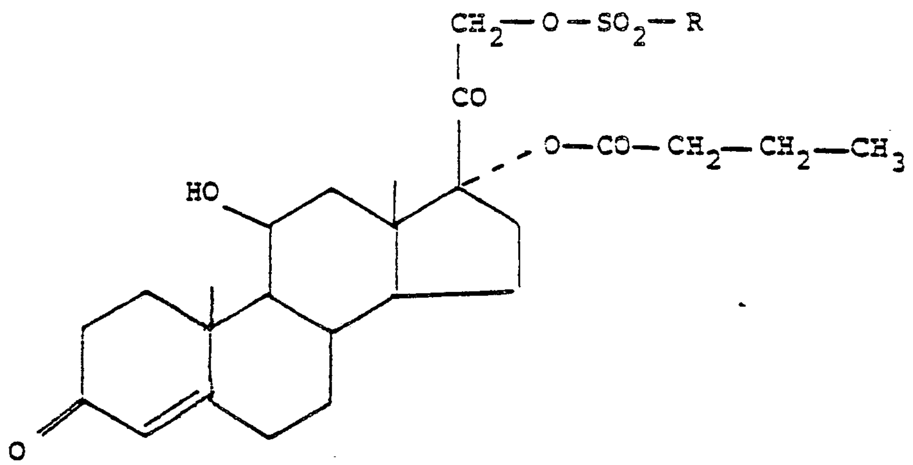

worin R einen niedrigen Alkylrest bedeutet mit einem Thiocarboxylat eines Alkalimetalls der Formel

$$M—S—CO—R_1$$

worin M ein Alkalimetallatom und $R_1$ Methyl oder Ethyl bedeutet, umsetzt.
4. Medikament, insbesondere ein entzündungshemmendes, dadurch gekennzeichnet, daß es eine der Verbindungen nach Anspruch 1 und Anspruch 2 enthält.

**Patentansprüche** (für den Vertragsstaat AT)

1. Verfahren zur Herstellung des S-21-Thioacetat-17-butyrat von 11β-Hydroxy-4-pregnen-3,20-dion und des S-21-Thiopropionat-17-butyrat von 11β-Hydroxy-4-pregnen-3,20-dion, dadurch gekennzeichnet, daß man ein 21-Sulfonat des Steroides der Formel

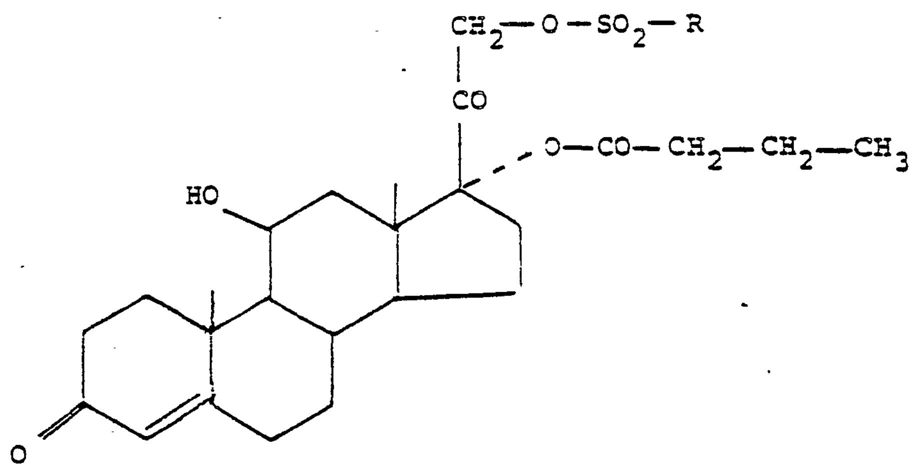

worin R einen niedrigen Alkylrest bedeutet mit einem Thiocarboxylat eines Alkalimetalls der Formel

$$M—S—CO—R_1$$

worin M ein Alkalimetallatom und $R_1$ Methyl oder Ethyl bedeutet, umsetzt.
2. Verfahren zur Herstellung einer pharmazeutischen Zubereitung, bei dem man das S-21-Thioacetat-17-butyrat von 11β-Hydroxy-4-pregnen-3,20-dion oder das S-21-Thiopropionat-17-butyrat von 11β-Hydroxy-4-pregnen-3,20-dion mit einem pharmazeutischen Trägerstoff oder Füllstoff vermischt.